# NEW EUROPEAN PATENT SPECIFICATION

(11) **EP 3 407 852 B2**
(45) Date of publication and mention of the opposition decision: **27.03.2024**
(45) Mention of the grant of the patent: 03.03.2021
(21) Application number: 17706011.8
(22) Date of filing: 27.01.2017
(51) Int. Cl.: A61J 1/10, A61J 1/14, B32B 27/08, B32B 27/30, B32B 27/32, A61J 1/16, B32B 27/16

(54) **BAG ASSEMBLY STERILIZABLE BY GAMMA IRRADIATION**
DURCH GAMMASTRAHLUNG STERILISIERBARE BEUTELANORDNUNG
ENSEMBLE SAC STÉRILISABLE PAR EXPOSITION À UN RAYONNEMENT GAMMA

(30) Priority: 29.01.2016 US 201662289028 P
(43) Date of publication of application: 05.12.2018
(60) Divisional application: 20215155.1
(73) Proprietor: Entegris, Inc., Billerica, MA 01821 (US)
(72) Inventor: SCHLEICHER, Michael, J., Billerica MA 01821 (US); BROSCH, Brenna, Chanhassen MN 55403 (US); TOLLESON, Alex, Minneapolis MN 55403 (US); KOLAND, Amy, Eden Prairie MN 55347 (US); JOHNSON, Michael, W., Billerica MA 01821 (US); LEYS, John, A., Billerica MA 01821 (US)
(74) Representative: Greaves Brewster LLP
(86) International application number: PCT/US2017/015397
(87) International publication number: WO 2017/132543

(56) References cited:
- US-A- 5 911 340
- US-A1- 2006 246 244
- US-A1- 2006 246 244
- US-A1- 2008 199 645
- US-A1- 2011 097 563
- US-A1- 2012 284 991
- US-B1- 7 207 153
- US-B2- 8 273 070
- MDS Nordion: "Gamma Compatible Materials Reference Guide", Nordion Nordion, 1 August 2007 (2007-08-01), pages 1-4, Retrieved from the Internet: URL:https://www.nordion.com/wp-content/upl oads/2019/01/Gamma_Compatible_Brochure_201 9-v2.pdf [retrieved on 2021-12-17]
- Anonymous: "Radiation Sterilization-Material Qualification", Association for the Advancement of Medical Instrumentation Technical Information Report AAMI TIR 17-97, 1 January 1997 (1997-01-01), pages 1-55,
- Rosenberg Y., Siegmann A., Narkis M., Shkolnik S.: "Low dose ?-irradiation of some fluoropolymers: Effect of polymer chemical structure", Journal of Applied Polymer Science, John Wiley & Sons, Inc., US, vol. 45, no. 5, 15 June 1992 (1992-06-15), pages 783-795, US ISSN: 0021-8995, DOI: 10.1002/app.1992.070450504

## Description

### TECHNICAL FIELD

This disclosure relates to a bag assembly that is capable of withstanding sterilization by gamma irradiation, and more particularly to a fluoropolymer bag assembly that is cable of withstanding sterilization by gamma irradiation.

### BACKGROUND

Chemical, biological, and biopharmaceutical manufacturing facilities have traditionally employed the use of large stainless-steel vats in their manufacturing processes. Due to the expense of these systems, including the costs involved in cleaning and sterilizing vats between production batches, manufacturers are increasingly moving toward single-use systems for holding chemical or biological media during processing, storage, and/or transport of the media. US 2011/097563 describes medical containers which are made of a multi-layered film including a polyolefin and which can be sterilised by autoclave. US 2006/246244 describes a sterile disposable bag with an interior surface made of melt-fabricable fluoropolymer.

### SUMMARY

The present disclosure relates generally to robust bag assemblies that are capable of withstanding sterilization by gamma irradiation, and securely retaining a chemical, biological, and biopharmaceutical media, while also allowing for easy access to the interior of the bag assemblies for the filling and extraction of the media from the bag assembly.

According to various embodiments, a bag assembly capable of withstanding sterilization by gamma irradiation includes a bag portion and a fitment. The bag assembly also includes at least one fitting extending from the fitment, wherein the at least one fitting is in communication through the fitment to the interior of the bag portion. The bag assembly can be a stand-alone article or can be contained within an outer container. The bag assembly is capable of withstanding an amount of gamma radiation of: at least 50 kGy of gamma radiation up to about 100 kGy of gamma radiation, to at least 50 kGy of gamma radiation up to about 200 kGy of gamma radiation, at least 50 kGy of gamma radiation up to about 500 kGy of gamma radiation; or at least 50 kGy of gamma radiation up to about 1000 kGy of gamma radiation. In addition to its ability to withstand sterilization by gamma irradiation, the bag assembly, in certain embodiments, may withstand temperatures of -150 degrees C or even lower as determined by ISO 8570 cold crack test. Some or the disclosed bag assemblies have a water vapor permeability of less than 5 g/m2.d.bar. Others are UV stable. Some of the disclosed bag assembly may be desirably formed without the use of adhesives, solvents, or binders.

The bag portion of the bag assembly includes first and second walls, wherein each of the first and second walls comprise a single sheet of a single layer fluoropolymer film comprising an ethylenetetrafluoroethylene polymer. The fitment includes first and second sidewalls extending between opposing end points, and can also be composed of a fluoropolymer. A portion of each of the first and second walls of the bag portion are attached to each other along at least a portion of a perimeter of the bag assembly up to the opposing end points of the fitment defining an interior of the bag portion. In some embodiments, the first and second walls can be continuously bonded along a majority of the perimeter of the bag assembly. In addition, each of the first and second walls are bonded individually to one of the first and second sidewalls of the fitment.

In some embodiments, at least one of the first and second sidewalls of the fitment has a curved portion extending between the opposing end points. In other embodiments, each of the first and second sidewalls of the fitment have curved portions extending between the opposing end points. The fitment, in some aspects, can be composed of the same fluoropolymer as the fluoropolymer film used to form the first and second walls of the bag portion. The utilization of similar fluoropolymers may enhance the attachment between the first and second walls of the bag portion to the sidewalls of the fitment. One or more of the first and second walls of the bag portion, the fitment or the at least one fitting can be composed of an ethylenetetrafluoroethylene polymer, a polychlorotrifluoroethylene polymer, a polyvinyl fluoride polymer, a polyvinylidene fluoride polymer, or a combination thereof. In some embodiments, the fluoropolymer is an ethylenetetrafluoroethylene polymer.

According to various embodiments, a method includes: (a) producing a bag assembly having first and second walls by providing at least one sheet of a single layer fluoropolymer film comprising an ethylenetetrafluoroethylene polymer, a fitment including first and second sidewalls extending between opposing end points, and at least one fitting extending from the fitment and in communication through the fitment to the interior of the bag portion; (b) attaching a portion of each of the first and second walls to each other along at least a portion of a perimeter of the bag assembly up to the opposing end points of the fitment, and (c) attaching each of the first and second walls individually to one of the first and second sidewalls of the fitment to form an internal enclosure, wherein the at least one fitting is in communication through the fitment to the internal enclosure, and wherein the bag assembly is capable of withstanding sterilization by gamma irradiation. Additionally, the method includes irradiating the bag assembly with at least 50 kGy of gamma radiation up to 1000 kGy of gamma radiation. In some embodiments, the bag assembly can be exposed to at least 50 kGy of gamma irradiation up to about 100 kGy of gamma radiation, to at least 50 kGy of gamma radiation up to about 200 kGy of gamma radiation, at least 50 kGy of gamma radiation up to about 500 kGy of gamma radiation; or at least 50 kGy of gamma radiation up to about 1000 kGy of gamma radiation.

In some embodiments, the method of attaching a portion of the first and second walls to each other and then individually to the first and second sidewalls of the fitment can include heat bonding or ultrasonic welding.

The preceding summary is provided to facilitate an understanding of some of the innovative features unique to the present disclosure and is not intended to be a full description. A full appreciation of the disclosure can be gained by taking the entire specification, claims, drawings, and abstract as a whole.

### BRIEF DESCRIPTION OF THE DRAWINGS

The disclosure may be more completely u̅n̅d̅e̅r̅stood in consideration of the following description of various illustrative embodiments in connection with the accompanying drawings.
FIG. 1A is an elevational view of a side of a bag assembly according to an embodiment of the disclosure.
FIG. 1B is an enlarged view of a top portion (detail A) of the bag assembly of FIG. 1A.
FIG. 1C is an elevational view of the bottom of the bag assembly of FIG. 1A.
FIG. 1D is a perspective view of the bag assembly of FIG. 1A.
FIG. 2A is a perspective view of a fitment according to an embodiment of the disclosure.
FIG. 2B is an elevational view of a top of the fitment of FIG. 2A.
FIG. 2C is an elevational view of a bottom of the fitment of FIG. 2A.
FIG. 2D is an elevational view of a side of the fitment of FIG. 2A.
FIG. 2E is a cross-sectional view of a port taken along line A-A of FIG. 2D.
FIG. 2F is an enlarged view of a port (detail C) of FIG. 2D.
FIG. 3A is a perspective view of a fitment according to another embodiment of the disclosure.
FIG. 3B is a cross-sectional view of the fitment of FIG. 3A.
FIG. 4 is a perspective view of a bag assembly contained within an outer container according to an embodiment of the disclosure.
FIG. 5 is a perspective view of the outer container of FIG. 4 with the lid in a closed position.

While the disclosure is amenable to various modifications and alternative forms, specifics thereof have been shown by way of example in the drawings and will be described in detail. It should be understood, however, that the intention is not to limit aspects of the disclosure to the particular illustrative embodiments described. On the contrary, the intention is to cover all modifications, equivalents, and alternatives falling within the scope of the disclosure.

### DESCRIPTION

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise. As used herein, the term "about" refers to a range of numbers that is considered equivalent to the recited value (e.g., having the same function or result). In many instances, the term "about" may include numbers that are rounded to the nearest significant figure.

As used herein, the term "attached" is generally used to refer two or more structures that are joined, fastened or otherwise connected to one another. In some cases, the term "attached" is used herein to refer to two or more structures that have been bonded or welded together. More particularly, the term "attached" is used herein to refer to two or more structures that have been bonded or welded together without the use of adhesives, solvents or binders.

As used herein, the term "gamma radiation" is defined as penetrating electromagnetic radiation of a kind arising from the radioactive decay of atomic nuclei. Gamma radiation is composed of photons in the highest observed range of photon energy. The effect of gamma radiation on a material is more closely related to the amount of energy deposited rather than the charge, and is referred to as the absorbed dose. The gray (Gy), which has units of joules per kilogram (J/kg), is the SI unit of absorbed dose, and is the amount of radiation required to deposit 1 joule of energy in 1 kilogram of any kind of matter.

As used herein, the terms "gamma sterilization" or "gamma irradiation" refers to the use of gamma radiation to sterilize an article. Gamma sterilization or gamma irradiation uses Cobalt 60 to kill microorganisms on a variety of different products. The radioisotope Cobalt 60 is the energy source for use in gamma irradiation with the irradiation process taking place in a specially designed cell. A characteristic of gamma irradiation is its high penetration capability.

As used herein, the term "capable of withstanding sterilization by gamma irradiation" is used to refer to a material that does not substantially degrade, physically or chemically, when exposed to gamma radiation.

As used herein, the term "materially stable" refers to a material or assembly that substantially maintains its mechanical and chemical integrity when subjected to gamma radiation.

As used herein, the terms "UV stable" or "UV stability" is used to describe a material that does not substantially degrade when exposed to ultraviolet radiation.

As used herein, the term "single-use" encompasses a broad range of primarily plastic disposable technologies that are suitable for a wide variety of scales and applications, from upscale bioprocessing to final formulation and filling.

As used herein, the term "fitment" refers to a structure that provides communication with the interior of a bag assembly and that facilitates the introduction of media or the removal of media from the interior of the bag assembly.

As used herein, the term "fitting" refers to a structure that is connectable to the fitment, and which also provides communication with the interior of the bag assembly. A fitting typically includes additional structural features which facilitates a connection between different structures. Exemplary fittings include, but are not limited to hose barbs, Luer fittings, quick connectors, such as Primelock^{®} (Entegris, Billerica, MA) and Quikgrip^{®} fittings (Entegris, Billerica, MA), and flare-type fittings, such as Flaretek^{®} fittings (Entegris, Billerica, MA).

As used herein, the term "two-dimensional bag assembly" refers to a bag assembly having a bag portion in which one or more sheets of a polymer film are welded or bonded together at their edges to form a generally flat, pillow-like enclosure defining an interior.

As used herein, the term "outer container" is used to refer to a first container in which is disposed a second container such as, for example, a bag assembly as described herein. In some cases, the outer container can be rigid and can protect the inner container or bag assembly, but this is not required.

As used herein, the term "single layer" refers to a sheet having a single layer of a polymer material and excludes any intervening layers such as barrier layers, adhesive layers tie layers, or combinations thereof.

As used herein, the term "polymer" refers to substance that has a molecular structure consisting chiefly or entirely of a large number of similar units, called monomer units, bonded together.

As used herein, the term "homopolymer" refers to a polymer which consists of repeating, identical monomer units.

As used herein, the term "copolymer" refers to a polymer made by reaction of two different monomers, with units of more than one kind.

The following detailed description should be read with reference to the drawings in which similar elements in different drawings are numbered the same. The detailed description and the drawings, which are not necessarily to scale, depict illustrative embodiments and are not intended to limit the scope of the disclosure. The illustrative embodiments depicted are intended only as exemplary. Selected features of any illustrative embodiment may be incorporated into an additional embodiment unless clearly stated to the contrary.

Chemical, biological, and biopharmaceutical materials often require storage in vessels, such as polymeric containers, that are intended to offer a stable and sterile environment. However, such storage vessels may undesirably contaminate the material contained in the vessel by leaching chemicals or other extractables from the materials forming the vessel or through contaminants remaining in the vessel. Additionally, attempts to sterilize certain vessels may adversely affect the materials of construction and degrade those materials. The unwanted degradation can possibly introduce new contaminants and severely limit the service life or potential application of the vessel.

The bag assemblies, as described herein according to the various embodiments, overcome some of the challenges of currently available bag assemblies by providing a product that possesses one or more desirable characteristics such as for example a high purity; chemically compatible with a variety of media; able to be used in cold storage and more particularly, cryogenic applications; and is materially stable when sterilized. Sterilization may be accomplished by exposing the bag assembly to gamma rays. Without being bound by theory, it is believed gamma irradiation is capable of killing bacteria by breaking the covalent bonds of bacterial DNA. Gamma irradiation can be one effective form of sterilization and advantageous because gamma irradiation is not a heat-generating process. Heat generated during certain sterilization practices can stress the bag assembly and could compromise the stability and durability of the completed article. Gamma irradiation also does not involve the use of moisture and, as such, does not require drainage of condensation or outgassing following sterilization. Additionally, in some embodiments, following gamma irradiation, there is substantially no residual radioactivity detected from the bag.

According to various embodiments, the disclosed bag assembly can be constructed from a fluoropolymer film that is selected for a combination of chemical and physical properties. Non-limiting properties of the fluoropolymer film may include the ability to withstand sterilization by gamma irradiation; processability at high shear rates; low water vapor permeability; chemical compatibility with a wide range of materials; UV stability; and the ability to withstand temperatures of -150 °C, possibly -190 °C, or possibly lower. In certain embodiments, the bag assembly is capable of withstanding up to of at least 50 kGy and up to about 100 kGy, 200 kGy, 500 kGy, or 1000 kGy of gamma radiation.

The bag portion of the bag assembly is generally constructed of a fluoropolymer film. The fluoropolymer film includes an ethylenetetrafluoroethylene (ETFE) polymer. ETFE isparticularly suited for construction of the bag assemblies. In accordance with this disclosure, certain fluoropolymer films may desirably possess one or more of (i) the ability to withstanding sterilization by gamma irradiation, (ii) may be processed at high shear rates, (iii) are melt processable for example by injection-molding, (iv) possess chemically stability in the presence of a wide range of materials, (v) withstand cold temperatures including those as low as -150° C and more particularly, as low as -190 °C when subjected to cold crack testing as specified by ISO 8570:1991 (E), (vi) have a water vapor permeability of 2 g/m².d.bar, and is UV stable.

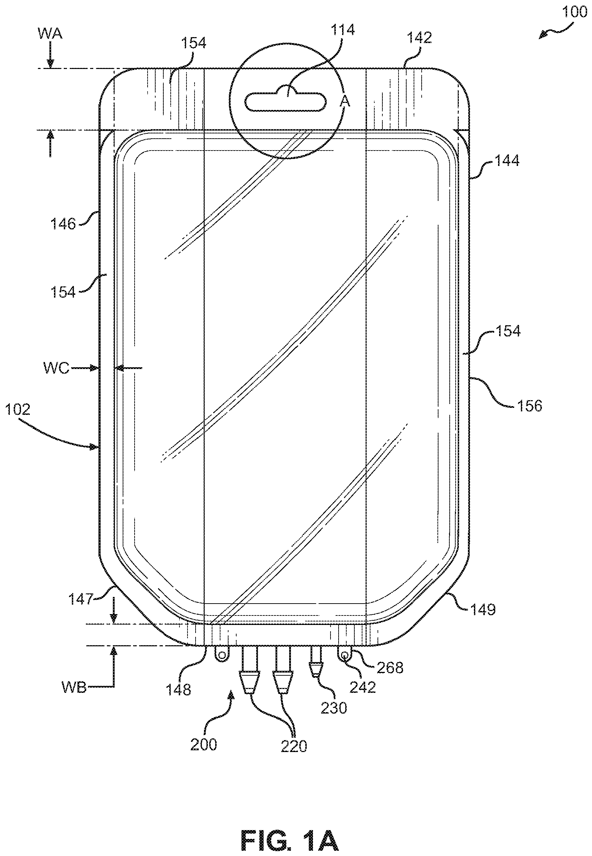

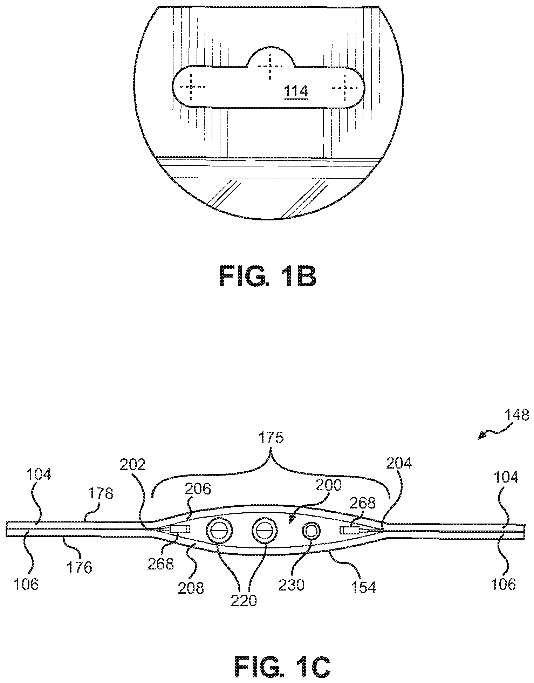

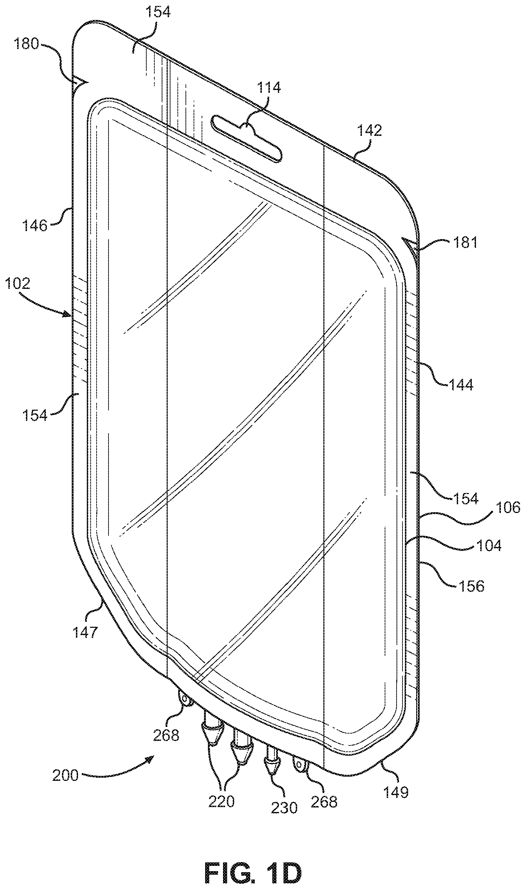

Turning now to the drawings, FIGS. 1A-1D show an exemplary bag assembly 100. As best seen in FIGS. 1A and ID, the bag assembly 100 includes a bag portion 102 and a fitment 200. In some cases, the first and second walls 104, 106 are welded or bonded together at their respective edges along a majority of the perimeter 156 of the bag assembly 100 up to the opposing ends 202, 204 of the fitment 200 (FIG 1C) to define the interior of the bag portion 102. The bond or weld formed between the first and second walls 104, 106 can be a continuous bond or weld about a majority of the perimeter 156 of the bag assembly 100 up to the opposing ends 202, 204 of the fitment 200. Any suitable welding or bonding technique known to those of skill in the art may be used to attach the first and second walls 104, 106 of the bag portion together at their respective edges 142, 144, 146, 147 and 149. Exemplary welding or bonding techniques can include, but are not limited to heat bonding, impulse welding, laser welding, ultrasonic welding, platen welding, or the like. However, in many cases, the first and second walls 104, 106 of the bag portion 102 are attached to each other at their respective edges without the use of adhesives, solvents or binders. Eliminating the use of adhesives, solvents or solvents in the construction of the bag assembly 100 can enhance the overall purity of the final assembly as the number of sources of potential leachable and extractables are reduced.

In some embodiments, as shown in FIG. 1A, the bag portion 102 includes first and second walls 104, 106, a top edge 142, two side edges 144 and 146, two angled side edges 147 and 149, and a bottom edge 148. The first and second walls 106, 104 can be attached to each other at the respective edges 142, 144, 146, 147 and 149 of the bag portion 102 to form a seam 154 along at least a portion of the perimeter 156 of the bag assembly 102 to define an interior of the bag portion 102. As depicted in FIG. 1A, seam 154 of bag assembly 100 can have varying seam widths along the perimeter of the bag. The width of seam 154 can provide a sufficient weld width or weld thickness for structural durability as well as providing expanded areas to accommodate additional features, such as opening 114 through which a hook or other fixture can be threaded such that the bag assembly 100 can be suspended. For example, the seam 154 can be formed to have a weld width WA along top edge 142, weld width WB along bottom edge 148, and weld width WC around side and angled edges 144, 146, 147, 149. Weld width WB can be made to accommodate the size of fitment 200, and to provide a sufficiently large weld width to effectively bond films 176, 178 to the fitment 200. Weld width WC can be at least a minimum weld width to provide an effective seal along seam 154. Weld width WA can be made wider to provide additional area for opening 114. In addition, the angled side edges 147, 149 can reduce stress concentrations that are typically formed with sharp corners. The angled side edges 147, 149, as opposed to right-angled edges near bottom edge 148, can provide a more balanced distribution of weight of media contained in the bag assembly 100, decreasing strain on seam 154 along edges at or near the bottom of the bag.

In some embodiments, the first and second walls 104, 106 are heat bonded to each other and to the fitment over a weld width, or weld thickness, of about 1/8 inch (about 3.175 mm) to about 1/2 inch (about 12.7 mm). In a particular embodiment, the first and second walls are heat bonded to each other and to the fitment over a weld width of about 1/4 inch (about 6.35 mm). A separate weld can be used along edge 142, with this weld being a different width than the weld provided around edges 144, 146, 147-149. This is shown in Figure ID, for example, where unsealed areas 180, 181 are located between the welds at two corners of bag assembly 100.

Each of the first and second walls 104, 106 of the bag portion 102 can be formed from a single sheet of a fluoropolymer film. In certain aspects, the first and second walls 104, 106 of the bag portion 102 are formed from a single sheet of a fluoropolymer film. The single sheet of fluoropolymer film excludes any intervening layers such as barrier layers, adhesive layers tie layers or combinations thereof. Use of a single sheet of a fluoropolymer film having no intervening layers reduces the potential number of sources of leachables or extractables, and may enhance the overall purity of the final assembly.

In one embodiment, each sheet of fluoropolymer forming the walls 104, 106 of the bag portion 102 of the bag assembly 100 can have a thickness of about 2.5 mil (63.5 p.m) to about 20 mil (508 p.m), or of about 5 mil (127 p.m) to about 15 mil (381 p.m). In one embodiment, each sheet of fluoropolymer film forming the walls lof a bag assembly has a thickness of about 8 mil (203.2 p.m).

As best viewed in FIG. 1C, the first and second walls 104, 106 of the body portion 102 are attached to the fitment 200 to form the bag assembly 100. The fitment 200 forming a part of the bag assembly 100 can be any suitable structure that provides communication with the interior of a bag assembly, and that facilitates the introduction or removal of a fluid or other substance such as, for example, a powdered material, from the interior of a bag assembly such as, bag assembly 100. As shown in FIG. 1C, the fitment 200 includes a first side wall 206 and a second side wall 208 extending between two opposing end points 202, 204. At least one of the first and second side walls 206, 208 can be curved. In some cases, both of the first and second side walls 206, 208 are curved between the opposing end points 202, 204 of the fitment. More particularly, both of the first and second side walls 206, 208 of the fitment can be curved such that the first and second side walls 206, 208 are symmetrical about a longitudinal axis of the fitment 200.

A portion of the first wall 104 is attached to a first side wall 206 of the fitment 200 and a portion of the second wall 106 of the bag portion 102 is attached to the second side wall of the fitment 200 thus placing the fitment 200 in communication with the interior of the bag portion 102. The first and second walls 104 of the bag portion 102 can be welded or bonded to the first and second side walls 206, 208 such that a continuous bond or weld is formed about the entire perimeter 156 of the bag assembly 100. In some cases, as can be seen in FIG. 1C, the first and second walls 104, 106 of the bag portion 102 are attached to first and second sidewalls 206, 208 of the fitment such that the walls follow the contour of the walls 206, 207 of the fitment 200. The contoured shape of the fitment 200 provides a continuous surface for the attachment of each of the first and second walls 104, 106 of the bag portion 102 to the respective sidewalls 206, 208 of the fitment 200. The attachment of the first and second walls 104, 106 along a continuous surface may remove any points of weakness that would occur if, for example, the walls were bonded to a fitment having sharper angled edges or to a fitment with a more circular shape, and may result in a more robust attachment between the bag portion 102 and the fitment 200. Any suitable bonding or welding technique can be used to attach the first and second walls 104, 106 of the bag portion 102 to the first and second sidewalls 206, 208 of the fitment 200. For example, the first and second walls 104, 106 of the bag portion 102 may be attached to the side walls 206, 208 of the fitment using heat boding, laser welding, ultrasonic welding, or platen welding techniques. In many embodiments, the attachment between the walls 104, 106 of the bag portion 102 and the side walls 206, 208 of the fitment 200 is made without the use of adhesives, solvents or binders, which can reduce the potential for leachables and extractables in the final bag assembly 100. In some embodiments, the same attachment method used to attach the edges of the first and second walls 102 together can be used to attach a portion of the first and second walls 104, 106 to the first and second side walls 206, 208 of the fitment 200.

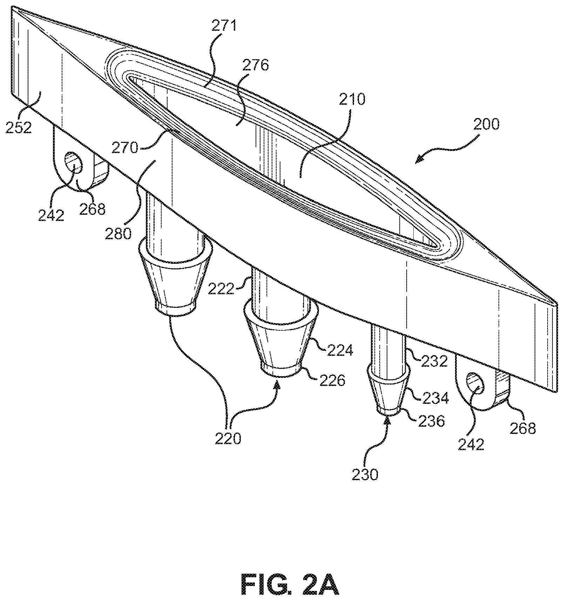

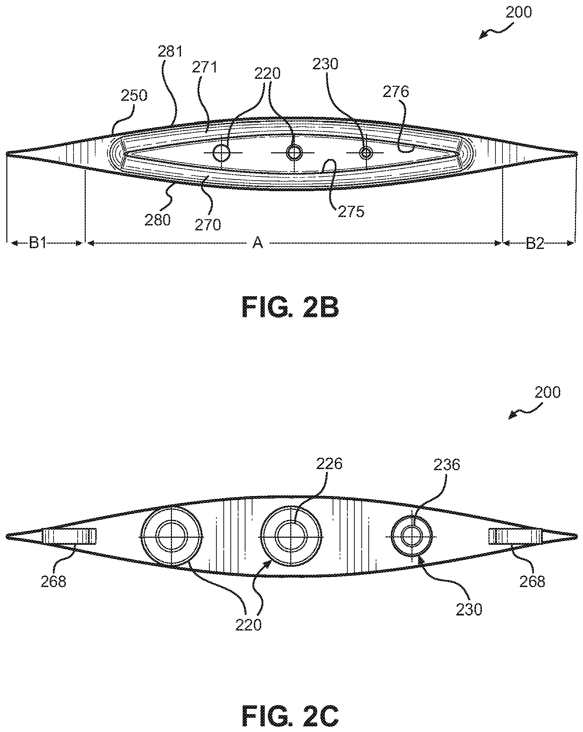

The fitment 200 is shown in further detail in FIGS. 2A-2E. In some embodiments, as best seen in FIG. 2B, the side walls 206, 208 of the fitment 200 are generally tapered from a convex region A, forming a central portion of the fitment 200, to slightly concave regions B1 and B2 located at either end of the fitment. The strength of an attachment formed between walls 204, 206 of the bag portion and the side walls 206, 208 (when the fitment is installed as part of a bag assembly) can be positively correlated with the radius of curvature of the surface of the fitment 200. The geometric shape of the sidewalls 206, 208 maximizes the bend radius and eliminates discontinuities, permitting a smooth seal to be formed with each of the respective the bottom edges 148 of the first and second walls 104, 106 of the bag portion 102 when the fitment 200 is installed as part of bag assembly 100. Those of ordinary skill in the art will recognize that the fitment may possess various shapes and dimensions that enable bonding of the fluoropolymer film to the fitment to form the disclosed bag assembly. For example, in some embodiments, the fitment 200 can be a boat-shaped fitment. Boat-shaped fitments are also referred to as cat's eye fitments and diamond-shaped fitments.

Like the walls 104, 106 of the bag portion 102, the fitment 200 also can be formed from a fluoropolymer. In some aspects the fluoropolymer is a melt processable polymer that may enable formation of the fitment using conventional molding techniques such as injection molding. The fitment 200 can be formed from the same or different fluoropolymer as the first and second walls 104, 106. The criteria for selecting the fluoropolymer used to form the fitment 200 may be the same criteria as that used to select the fluoropolymer film used to form the walls of the bag portion. The fluoropolymer from which the fitment 200 can be formed is selected for a combination of chemical and physical properties. The fitment may desirably possess one or more of (i) the ability to withstanding sterilization by gamma irradiation, (ii) may be processed at high shear rates, (iii) are melt processable for example by injection-molding, (iv) possess chemically stability in the presence of a wide range of materials, (v) withstand cold temperatures including those as low as -150° C and more particularly, as low as -190 °C when subjected to cold crack testing as specified by ISO 8570:1991 (E), (vi) have a water vapor permeability of 2 g/m².d.bar and is UV stable.

In some embodiments, the fluoropolymer used to form the fitment 200 includes an ethylenetetrafluoroethylene (ETFE) polymer, a polychlorotrifluoroethylene (PCTFE) polymer, a polyvinyl fluoride (PVF) polymer, a polyvinylidene fluoride (PVDF) polymer or a combination of these. In another embodiment, the fluoropolymer includes an ethylenetetrafluoroethylene (ETFE) polymer, a polyvinyl fluoride (PVF) polymer, a polyvinylidene fluoride (PVDF) polymer or a combination of these. In still another embodiment, ETFE is particularly suited for construction of the fitment for similar reasons as disclosed above for the fluoropolymer film.

The fitment 200 is configured with one or more fittings 220, 230 to enable ready and reliable connection with various parts and objects, such as tubing, connectors, hoses, syringes or the like. The fitment 200 includes at least one fitting 220, 230 that extend from the fitment 200, and is in communication through the fitment 200 to the interior defined by the walls of the bag portion 102. The fitting(s) 220, 230 facilitates connection of various hoses, tubing, connectors, syringes or the like to the bag assembly 100. Exemplary fittings include, but are not limited to, hose barbs, Luer fittings, quick connectors,
such as Primelock^{®} (Entegris, Billerica, MA) and Quikgrip^{®} fittings (Entegris, Billerica, MA), and flare-type fittings, such as Flaretek^{®} fittings (Entegris, Billerica, MA).

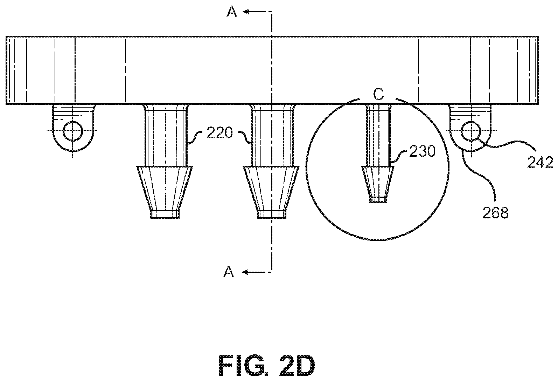

In some embodiments, as shown in FIG. 2A, the fitment 200 includes an opening 210, placing three fittings, hose barbs 220 and 230, in fluid communication with the interior of a bag assembly when the fitment 200 is included as part of the bag assembly 100. The fitment 200 and opening 210 can be sized to accommodate communication between the bag interior and a variable number of fittings, including fittings, such as the hose barbs shown 220 and 230 shown in FIGS. 2A and 2D. The various fittings 220 or 230 may be of the same size of different sizes. As illustrated, the fitment 200 includes three hose barbs, including one of a small barb 230 and two of a large barb 220, although other configurations are possible. In some embodiments, one of the two large barbs 220 can function as an inlet, the other of the two large barbs 220 can function as an outlet, and the small barb can function as a test port. Alternatively, the fitment 200 can include any combination of large or small fittings. For example, fitment 200 could include one large fitting, two large fittings, one large fitting and one small fitting, three large fitting, and any other combination of the number and size of fittings as would be contemplated by those of skill in the art. Additionally, the fitment 200 can include fittings of different types. For example, fitment 200 can include one hose barb and one Luer fitting, two hose barbs and one Flaretek^{®} fitting, or any other combination of fitting types.

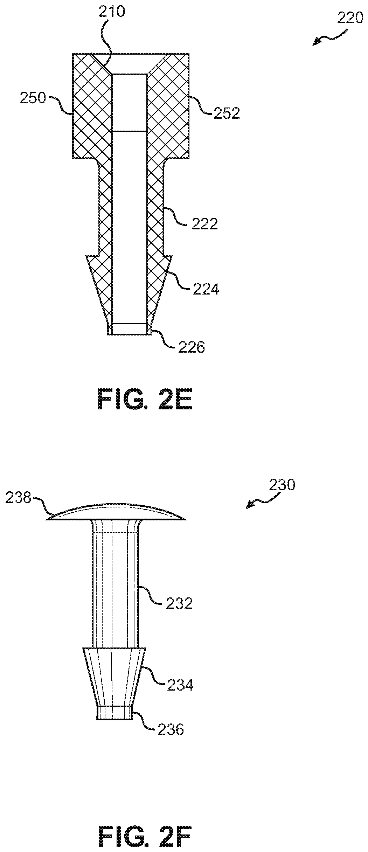

In some embodiments, one or more fittings such as, for example, fitting(s) 220 or 230 can be integrally formed with the fitment 200 such that the fitment 200 and the fitting 220 or 230 form a single, unitary structure. FIG. 2E is an expanded cross-sectional view of a large hose barb 220 that has been integrally formed with the fitment 200. As can be seen in FIG. 2E, the side walls 206, 208 of the fitment are continuous with a neck 222, tapered end region 224, and inlet/outlet 226 of the fitting 220.

In other cases, the fitting(s) 220 or 230 can be separate elements of fitment 200. When provided separately from the fitment 200, the fittings 220 or 230 can be inserted or otherwise connected to a port formed in the fitment 200. In such an embodiment, the fitting(s) 220 or 230 can be formed of the same or different fluoropolymer as the fitment 200. Preferably, the fitting is formed form the same fluoropolymer as the fitment 200. An example of a fitting that is provide as a component separate from a fitment is shown in FIG. 2F which depicts a hose barb fitting. As shown in FIG. 2F, hose barb fitting 230 includes a neck 232, tapered end region 234, and inlet/outlet 236. Hose barb fitting 230 can be fit or welded to a section 238 of the fitment 200.

In some embodiments, hose barb fittings 220, 230 can be used to provide exit and entry ports to the bag assembly 100. The inclusion of multiple barbs, such as the three-barb configuration shown in FIGS. 2A-2D, provides easy access for an end user of the bag, enabling multiple hose connections to either insert or withdraw media from the interior of the bag assembly. The inclusion of hose connections of multiple sizes, such as at least one large hose barb 220 and at least one small hose barb 230, enables flexibility and compatibility with other components of a packaging, storage, or processing system. In some embodiments, a bag assembly, such as disclosed herein, can include a fitment having at least one 0.25 inch (6.35 mm) hose barb and at least one 0.125 inch (3.175 mm) hose barb.

Referring again to Figures 2A and 2B, it can be seen that central opening 210 is enclosed by first and second inner sidewalls 270, 271. First inner sidewall 270 has an inner surface 275 and an outer surface 280 defining a first wall thickness. Similarly, second inner sidewall 271 has an inner surface 276 and an outer surface 281 defining a second wall thickness. Inner surfaces 275, 276 face central opening 210. The thickness of each sidewall 270, 271 is substantially constant along the length of fitment 200. In other words, the distance between inner surface 275 and outer surface 280 (or between inner surface 276 and outer surface 281) is substantially constant along the length of fitment 200. As a result, when fitment 200 cools after being heated during attachment of the walls 104, 106 of the bag portion 102 to the fitment 200, the fitment 200 is resistant to shrinking. The resistance of the fitment 200 to shrinking when cooled may facilitate formation of a more secure attachment between the fitment 200 and the walls 104, 106 during bonding or welding.

Additionally, in some embodiments, attachments can be provided as part of the fitment 200 to hold and align a fitment during the assembly process, including during attachment of walls 104, 106 to the sides walls 206, 208 of the fitment. For example, fitment 200 can include projections 268, shown in more detail in FIGS. 2A and 2D. Projections 268 include holes 242 through which a hook or other fixture can be threaded to hold, align, or suspend the fitment 200 during welding operations.

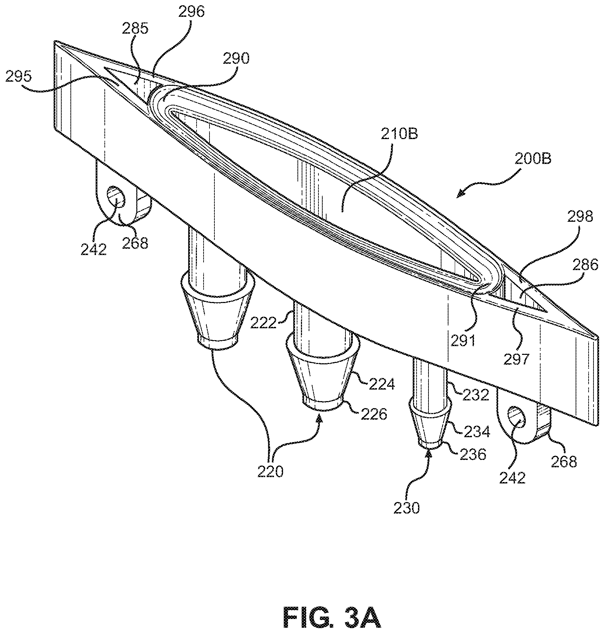

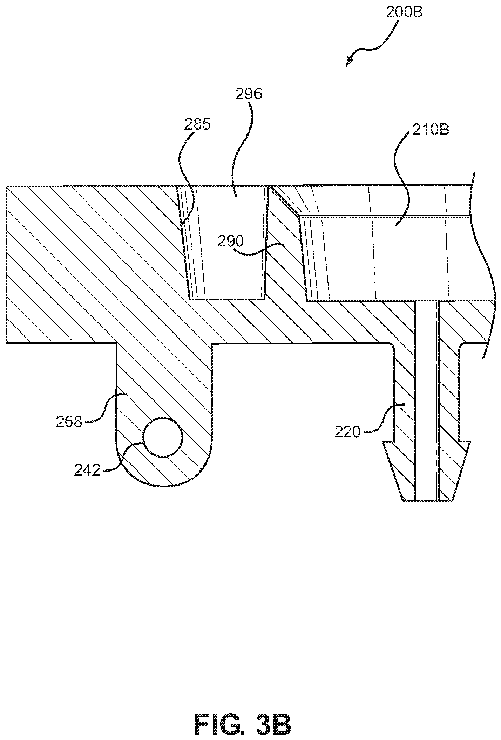

Figure 3A is a perspective view of a fitment 200B according to another embodiment of the disclosure. In particular, a fitment 200B is constructed in a substantially identical manner to fitment 200 except that the shape of an opening 210B is different than the shape of opening 210. In addition, fitment 200B includes cavities 285, 286, which are defined, in part, by ribs 290, 291. The inclusion of cavities 285, 286 reduces the amount of material present at the ends of fitment 200B relative to an otherwise identical fitment not including cavities 285, 286. Also, each of ribs 290, 291 and walls 295-298 preferably has a constant thickness such that fitment 200 resists shrinking when cooled, which may in turn facilitate formation of a more secure attachment between the fitment 200 and the walls 104, 106 during bonding or welding. This same result can also be accomplished by increasing the length of opening 210B. Such an arrangement is shown in FIGS. 2A and 2B. Specifically, opening 210 is longer than opening 210B such that opening 210 takes up a larger percentage of the length of fitment 200. Preferably, opening 210 extends along at least 75% of the length of fitment 200. Figure 3B is a cross-sectional view of fitment 200B showing cavity 285 and rib 290. Cavities 285, 286 are formed using corresponding pins (not shown) during molding of fitment 200.

A bag assembly, as described herein according to the various embodiments, can be formed without the use of adhesives, solvents or bonding. Eliminating the use of adhesives, solvents, or binders reduces the number of potential sources of leachables or extractables, which may enhance the overall purity of the final product. In some embodiments, the bag assembly can be formed by attaching a first sheet of a fluoropolymer film to a second fluoropolymer film at their edges about each of their respective perimeters to form the first and second walls of the bag portion of the bag assembly. In other embodiments, the bag assembly can be formed from one or more sheet of fluoropolymer film that can be folded to provide the first and second walls that are then attached to one another to form the first and second walls of the bag assembly. In either case, the first and second walls can be attached to one another at their edges about a majority of their respective perimeters, leaving a portion at a top or a bottom of the bag portion unattached such that a fitment can be inserted between the first and second walls of the fluoropolymer film. Next, the first and second walls can be separated from one another at the unattached portion such that a fitment is able to be inserted between the two walls. Once the fitment is inserted and properly positioned between the two sheets walls, each of the first and second walls of the fluoropolymer film can be individually attached to the corresponding sidewall of the fitment. The first and second walls of the fluoropolymer film can be attached to one another by any suitable attachment known to those of skill in the art including, but not limited to heat bonding,
impulse welding, platen welding, laser welding, ultrasonic welding or the like. The first and second walls of the film can be attached to the fitment using the same or a different attachment method. In one embodiment, a platen welding apparatus is used to attach the two walls to one another at their edges, and individually to each of the respective sidewalls of the fitment.

Once assembled, the bag assembly can undergo sterilization by gamma irradiation where the bag assembly can be subjected to at least 50 kGy and up to about 100 kGy, 200 kGy, 500 kGy, or 1000 kGy of gamma radiation.

The disclosed bag assembly may be utilized in various applications where the storage of the chemical, biological or biopharmaceutical materials require a stable and sterile environment that is capable of meeting strict physical demands. In various embodiments, the bag assemblies may possess one or more of (i) the ability to withstanding sterilization by gamma irradiation, (ii) possess chemically stability in the presence of a wide range of materials, (iii) withstand cold temperatures including those as low as -150° C and more particularly, as low as 190 °C when subjected to cold crack testing as specified by ISO 8570:1991 (E), (iv) have a water vapor permeability of 2 g/m².d.bar, and (v) are UV stable.

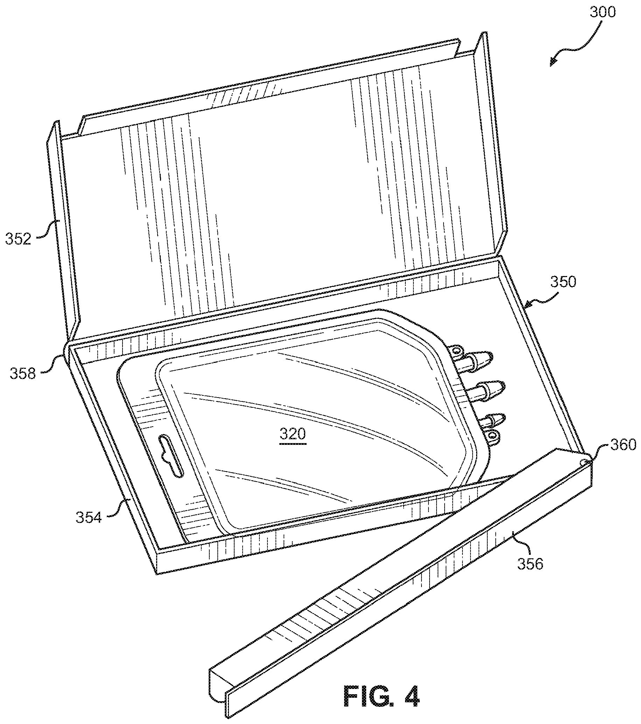

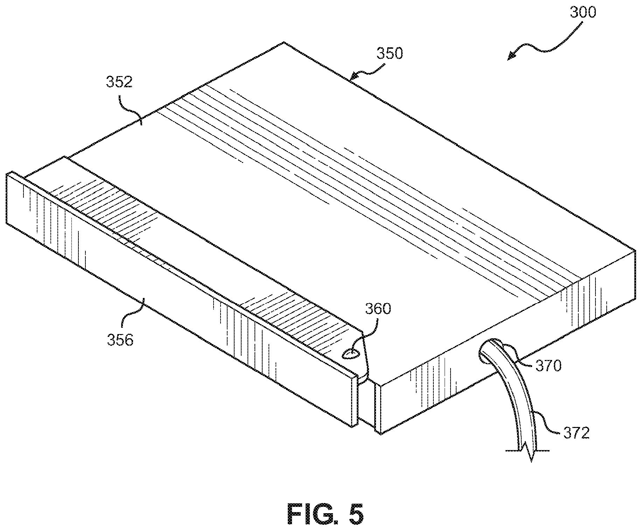

In some embodiments, a bag assembly, as described herein, can be placed within an outer container, such as a rigid clam-shell overpack or an overpack bag, for handling or storage. In one embodiment, a storage system includes a bag assembly contained within an outer container. An exemplary storage system 300 is shown in FIG. 4, with a bag assembly 320 contained within a rigid, clam-shell-type overpack 350. Bag assembly 320 can alternatively be contained within a soft overpack bag (not shown). The clam-shell overpack 350 is shown in FIG. 4 with a lid 352 in an open position from a bottom portion 354 that contains the bag assembly 320. Lid 352 and bottom portion 354 are connected by a hinge 358 located on one side of the overpack. A closure or locking mechanism 356 is attached to bottom portion 354 at hinge 360. Locking mechanism 356 swivels at hinge 360 to fasten and release lid 352. Overpack 350 is shown in FIG. 5 with lid 352 in a closed position and locking mechanism 356 fastened against lid 352. Alternative structures and mechanisms for closing an overpack about a liner are contemplated, other than hinge 358 and locking mechanism 356. For example, an overpack may be sealed shut. The overpack 350 can include one or more side ports 370 through which lines 372, such as tubing connected to the fitting(s) or port(s) of the bag, can extend so that the bag can still be contained within the overpack during use.

Overpacks can be fabricated from metal, rigid plastic, or a combination of both metal and plastic. The overpack protects a bag contained therein and can be used during storage, transport, and also during use of the bag itself. In addition, the overpack limits expansion of the bags during the freezing process. In various embodiments, the bag is a bag assembly or a single-use liner. In some cases, the bag may be a bag assembly that does not require the use of an outer container or overpack, such that it can be considered to be a stand-alone, bag assembly. In other cases, the bag may be a single-use liner configured to fit within an outer bag or an overpack. Bag assemblies and liners are intended to be discarded following their initial use.

### EXAMPLE

A 100mm X 100mm sample of an ethylenetetrafluoroethylene (ETFE) film having a single layer was subjected to cold crack testing per ISO 4593:1993(E). The film sampled in this example can be used in the formation of the bag assembly, as described herein.

The sample was prepared for cold crack testing per ISO 4593:1993(E), the steps of which included: cutting the 100mm X 100 sample into 60mm X 15mm strips; obtaining ten test strips for each temperature to be evaluated; bowing the strips in the form of a loop and mounting six of the strips in the cold crack test fixture; and conditioning the strips for four hours at 18°C +/-2°C per ISO 291:2008 (E). Cold crack test fixtures, such as those used in this example, are commercially available from a variety of manufactures. Following conditioning of the six strips, the test fixture was placed in a freezer at -50 °C until equilibrium was reached.

The test fixture was removed from the freezer, and the strips removed from the test fixture and visually inspected. The strips were determined to be broken or damaged if any one of the following imperfections were visually detected: trace of crack, points or dashes; trace of crack line; total rupture with no fragments; or total rupture with fragments.

If it was determined that none of the test strips were damaged or broken, the freezer temperature was then lowered by 5 °C and then the test was repeated with a set of new test strips. These steps were repeated until a temperature was reached at which 50% of the test strips were determined to be broken or damaged by visual inspection.

Following ISO 4593:1993(E) testing procedure, a single layer, ethylenetetrafluoroethylene (ETFE) film passed cold crack testing at -188 °C.

## Claims

1. An article comprising:
a bag assembly capable of withstanding sterilization by gamma irradiation of at least 50 kGy up to about 1000 kGy, the bag assembly including:
a bag portion having first and second walls, wherein each of the first and second walls comprise a single sheet of a single layer fluoropolymer film comprising an ethylenetetrafluoroethylene polymer; and
a fitment including first and second sidewalls extending between opposing end points, wherein a portion of each of the first and second walls of the bag portion are attached to each other along at least a portion of a perimeter of the bag assembly up to the opposing end points of the fitment defining an interior of the bag portion, and wherein each of the first and second walls are bonded individually to one of the first and second sidewalls of the fitment, and
at least one fitting extending from the fitment, wherein the at least one fitting is in communication through the fitment to the interior of the bag portion.

2. An article according to claim 1, wherein the first and second walls are continuously bonded along a majority of the perimeter of the bag assembly.

3. An article according to claim 1 or claim 2, wherein at least one of the first and second sidewalls of the fitment has a curved portion extending between the opposing end points.

4. An article according to any preceding claim, wherein each of the first and second sidewalls of the fitment have curved portions extending between the opposing end points.

5. An article according to any preceding claim, wherein the bag assembly comprises one or more of a cold cracking temperature of -150 degrees °C or lower as determined by ISO 8570 cold crack test, a water vapor permeability of less than 5 g/m²·d·bar, and is UV stable.

6. An article according to any preceding claim, wherein the fitment comprises a fluoropolymer.

7. An article according to any preceding claim, wherein the first and second walls of the bag portion and the fitment comprise the same fluoropolymer.

8. An article according to any preceding claim, wherein the bag assembly is formed in the absence of adhesives, solvents, binders or combinations thereof.

9. An article according to any preceding claim, wherein the fitment or the at least one fitting comprise an ethylenetetrafluoroethylene polymer, a polychlorotrifluoroethylene polymer, a polyvinyl fluoride polymer, a polyvinylidene fluoride polymer, or a combination thereof.

10. An article according to any preceding claim, wherein the bag assembly comprises a stand-alone article or is contained within an outer container.

11. A method comprising,
(a) producing a bag assembly having first and second walls by providing at least one sheet of a single layer fluoropolymer film comprising an ethylenetetrafluoroethylene polymer, a fitment including first and second sidewalls extending between opposing end points, and at least one fitting extending from the fitment and in communication through the fitment to the interior of the bag portion;
(b) attaching a portion of each of the first and second walls to each other along at least a portion of a perimeter of the bag assembly up to the opposing end points of the fitment, and
(c) attaching each of the first and second walls individually to one of the first and second sidewalls of the fitment to form an internal enclosure, wherein the at least one fitting is in communication through the fitment to the internal enclosure, and wherein the bag assembly is capable of withstanding sterilization by gamma irradiation; and
(d) irradiating the bag assembly with at least 50 kGy of gamma radiation up to 1000 kGy of gamma radiation.

## Patentansprüche

1. Gegenstand, umfassend:
eine Beutelanordnung, die der Sterilisation durch Gammabestrahlung von mindestens 50 kGy bis zu etwa 1000 kGy standhalten kann, wobei die Beutelanordnung umfasst:
einen Beutelabschnitt mit einer ersten und einer zweiten Wand, wobei jede der ersten und zweiten Wand eine einzige Folie eines ein Ethylenetetrafluorethylen-Polymer enthaltenden Einzelschicht-Fluorpolymerfilms umfasst, und
einen Einsatz mit einer ersten und einer zweiten Seitenwand, die sich zwischen gegenüberliegenden Endpunkten erstrecken, wobei ein Teil jeder der ersten und zweiten Wand des Beutelabschnitts entlang mindestens eines Teils eines Umfangs der Beutelanordnung bis zu den gegenüberliegenden Endpunkten miteinander verbunden ist und ein Inneres des Beutelabschnitts bildet, und wobei jede der ersten und zweiten Wand einzeln mit einer der ersten und der zweiten Seitenwand des Einsatzes verbunden ist, und
wenigstens ein Anschlussstück, das sich vom Einsatz erstreckt, wobei das wenigstens eine Anschlussstück über den Einsatz mit dem Inneren des Beutelabschnitts in Verbindung steht.

2. Gegenstand nach Anspruch 1, wobei die erste und die zweite Wand kontinuierlich entlang eines Großteils des Umfangs der Beutelanordnung verbunden sind.

3. Gegenstand nach Anspruch 1 oder Anspruch 2, wobei mindestens eine der ersten und zweiten Seitenwand des Einsatzes einen gekrümmten Abschnitt aufweist, der sich zwischen den gegenüberliegenden Endpunkten erstreckt.

4. Gegenstand nach einem der vorhergehenden Ansprüche, wobei jede der ersten und zweiten Seitenwand des Einsatzes einen gekrümmten Abschnitt aufweist, der sich zwischen den gegenüberliegenden Endpunkten erstreckt.

5. Gegenstand nach einem der vorhergehenden Ansprüche, wobei die Beutelanordnung eine oder mehrere Kältebruchtemperaturen von -150 °C oder weniger aufweist, bestimmt durch den Kältebruchtest nach ISO 8570, eine Wasserdampfdurchlässigkeit von weniger als 5 g/m²·d·bar aufweist und UV-stabil ist.

6. Gegenstand nach einem der vorhergehenden Ansprüche, wobei der Einsatz ein Fluorpolymer umfasst.

7. Gegenstand nach einem der vorhergehenden Ansprüche, wobei die erste und die zweite Wand des Beutelabschnitts und der Einsatz das gleiche Fluorpolymer umfassen.

8. Gegenstand nach einem der vorhergehenden Ansprüche, wobei die Beutelanordnung in Abwesenheit von Klebstoffen, Lösemitteln, Bindemitteln oder Kombinationen davon gebildet ist.

9. Gegenstand nach einem der vorhergehenden Ansprüche, wobei der Einsatz oder das wenigstens eine Anschlussstück ein Ethylenetetrafluorethylen-Polymer, ein Polychlortrifluorethylen-Polymer, ein Polyvinylfluorid-Polymer, ein Polyvinylidenfluorid-Polymer oder eine Kombination davon umfasst.

10. Gegenstand nach einem der vorhergehenden Ansprüche, wobei die Beutelanordnung einen eigenständigen Gegenstand umfasst oder in einem Außenbehälter enthalten ist.

11. Verfahren, umfassend
(a) Herstellen einer Beutelanordnung mit einer ersten und einer zweiten Wand durch Bereitstellen mindestens einer Folie eines ein Ethylenetetrafluorethylen-Polymer enthaltenden Einzelschicht-Fluorpolymerfilms, eines Einsatzes mit einer ersten und einer zweiten Seitenwand, die sich zwischen gegenüberliegenden Endpunkten erstrecken, und wenigstens eines Anschlussstücks, das sich vom Einsatz erstreckt, wobei das wenigstens eine Anschlussstück über den Einsatz mit dem Inneren des Beutelabschnitts in Verbindung steht,
(b) Befestigen eines Teils jeder der ersten und zweiten Wand aneinander entlang mindestens eines Teils eines Umfangs der Beutelanordnung bis zu den gegenüberliegenden Endpunkten des Einsatzes und
(c) Befestigen jeder der ersten und zweiten Wand einzeln an einer der ersten und zweiten Seitenwand des Einsatzes, um eine Inneneinschließung zu bilden, wobei das wenigstens eine Anschlussstück über den Einsatz mit der Inneneinschließung in Verbindung steht, und wobei die Beutelanordnung der Sterilisation durch Gammabestrahlung standhalten kann, und
(d) Bestrahlen der Beutelanordnung mit mindestens 50 kGy Gammastrahlung bis zu 1000 kGy Gammastrahlung.

## Revendications

1. Article comprenant :
un ensemble de poche capable de résister à la stérilisation par irradiation gamma d'au moins 50 kGy jusqu'à environ 1000 kGy, l'ensemble de poche comprenant :
une partie de poche ayant des première et seconde parois, dans lequel chacune des première et seconde parois comprend une feuille unique d'un film de polymère fluoré monocouche comprenant un polymère d'éthylène tétrafluoroéthylène ; et
un insert comprenant des première et seconde parois latérales s'étendant entre des points d'extrémité opposés, dans lequel une partie de chacune des première et seconde parois de la partie de poche sont attachées l'une à l'autre le long d'au moins une partie d'un périmètre de l'ensemble de poche jusqu'aux points d'extrémité opposés de l'insert définissant un intérieur de la partie de poche, et dans lequel chacune des première et seconde parois sont collées individuellement à l'une des première et seconde parois latérales de l'insert, et
au moins un raccord s'étendant à partir de l'insert, dans lequel l'au moins un raccord est en communication par le biais de l'insert avec l'intérieur de la partie de poche.

2. Article selon la revendication 1, dans lequel les première et seconde parois sont collées en continu le long d'une majorité du périmètre de l'ensemble de poche.

3. Article selon la revendication 1 ou la revendication 2, dans lequel au moins l'une des première et seconde parois latérales de l'insert a une partie courbe s'étendant entre les points d'extrémité opposés.

4. Article selon l'une quelconque des revendications précédentes, dans lequel chacune des première et seconde parois latérales de l'insert ont des parties courbes s'étendant entre les points d'extrémité opposés.

5. Article selon l'une quelconque des revendications précédentes, dans lequel l'ensemble de poche comprend une ou plusieurs parmi une température de fissuration à froid de -150 degrés C ou moins telle que déterminée par le test de fissuration à froid ISO 8570, une perméabilité à la vapeur d'eau inférieure à 5 g/m²•d•bar, et est stable aux UV.

6. Article selon l'une quelconque des revendications précédentes, dans lequel l'insert comprend un polymère fluoré.

7. Article selon l'une quelconque des revendications précédentes, dans lequel les première et seconde parois de la partie de poche et de l'insert comprennent le même polymère fluoré.

8. Article selon l'une quelconque des revendications précédentes, dans lequel l'ensemble de poche est formé en l'absence d'adhésifs, de solvants, de liants ou de combinaisons de ceux-ci.

9. Article selon l'une quelconque des revendications précédentes, dans lequel l'insert ou l'au moins un raccord comprend un polymère d'éthylène tétrafluoroéthylène, un polymère de polychlorotrifluoroéthylène, un polymère de polyfluorure de vinyle, un polymère de polyfluorure de vinylidène ou une combinaison de ceux-ci.

10. Article selon l'une quelconque des revendications précédentes, dans lequel l'ensemble de poche comprend un article autonome ou est contenu dans un contenant extérieur.

11. Procédé comprenant :
(a) la production d'un ensemble de poche ayant des première et seconde parois en fournissant au moins une feuille d'un film de polymère fluoré monocouche comprenant un polymère d'éthylène tétrafluoroéthylène, un insert comprenant des première et seconde parois latérales s'étendant entre des points d'extrémité opposés, et au moins un raccord s'étendant depuis l'insert et en communication par le biais de l'insert avec l'intérieur de la partie de poche ;
(b) l'attache d'une partie de chacune des première et seconde parois l'une à l'autre le long d'au moins une partie d'un périmètre de l'ensemble de poche jusqu'aux points d'extrémité opposés de l'insert, et
(c) l'attache de chacune des première et seconde parois individuellement à l'une des première et seconde parois latérales de l'insert pour former une enceinte interne, dans lequel l'au moins un raccord est en communication par le biais de l'insert avec l'enceinte interne, et dans lequel l'ensemble de poche est capable de résister à la stérilisation par irradiation gamma ; et
(d) l'irradiation de l'ensemble de poche avec au moins 50 kGy de rayonnement gamma jusqu'à 1000 kGy de rayonnement gamma.
